# EUROPEAN PATENT SPECIFICATION

(11) **EP 3 593 131 B1**
(45) Date of publication and mention of the grant of the patent: **17.02.2021**
(21) Application number: 18714605.5
(22) Date of filing: 06.03.2018
(51) Int. Cl.: G01N 33/28

(54) **SYSTEM FOR MONITORING OIL CONCENTRATION IN A LUBRICOOLANT MIXTURE OF A MACHINE TOOL**
SYSTEM ZUR ÜBERWACHUNG DER ÖLKONZENTRATION IN EINEM KÜHLSCHMIERSTOFFGEMISCH EINER WERKZEUGMASCHINE
SYSTÈME POUR SURVEILLER LA CONCENTRATION D'HUILE D'UN MÉLANGE LUBRIFIANT DE REFROIDISSEMENT DE MACHINE-OUTIL

(30) Priority: 06.03.2017 IT 201700024634
(43) Date of publication of application: 15.01.2020
(73) Proprietor: Unilock Di Umberto Crosti E C. S.A.S., 20090 Trezzano sul Naviglio (MI) (IT)
(72) Inventor: CROSTI, Umberto, 20090 Trezzano sul Naviglio (MI) (IT); GRASSI, Valerio, 20090 Trezzano sul Naviglio (MI) (IT)
(74) Representative: Perani & Partners S.p.A.
(86) International application number: PCT/IB2018/051433
(87) International publication number: WO 2018/163063

(56) References cited:
- US-A- 5 224 051
- KRISTÍNA GERULOVÁ ET AL: "REAL TIME MONITORING AND AUTOMATIC REGULATION SYSTEM FOR METALWORKING FLUIDS", RESEARCH PAPERS FACULTY OF MATERIALS SCIENCE AND TECHNOLOGY SLOVAK UNIVERSITY OF TECHNOLOGY, vol. 24, no. 38, 4 April 2016 (2016-04-04) , pages 27-34, XP055414869, DOI: 10.1515/rput-2016-0035 cited in the application
- Marco Grossi ET AL: "OIL CONCENTRATION MEASUREMENT IN METALWORKING FLUIDS BY OPTICAL SPECTROSCOPY *", Procedia Environmental Science, Engineering and Management, 5 November 2014 (2014-11-05), XP055414205, Retrieved from the Internet: URL:http://www.procedia-esem.eu/pdf/issues /2014/no1/3_Grossi_14.pdf [retrieved on 2017-10-10] cited in the application
- DATABASE COMPENDEX [Online] ENGINEERING INFORMATION, INC., NEW YORK, NY, US; February 1992 (1992-02), CARDUNER K R ET AL: "Determination of oil concentration in soluble oil cutting fluids using near-infrared spectroscopy", XP002781437, Database accession no. EIX92050479588 & CARDUNER K R ET AL: "Determination of oil concentration in soluble oil cutting fluids using near-infrared spectroscopy", PROCESS CONTROL AND QUALITY 1992 FEB, vol. 2, no. 2, February 1992 (1992-02), pages 143-154,

## Description

### Technical field

The present invention relates to a system for monitoring an oil concentration in a lubricoolant mixture for a machine tool, as defined in the preamble of claim 1.

Therefore, the present invention preferably but non exclusively concerns the field of metalworking by machine tools.

### Background art

Computer numerical control machine tools commonly used for metalworking must be lubricated for proper operation and cooled to prevent the high temperatures that inevitably occur during the working cycles from damaging the machine and affecting its efficiency. For this purpose, fluid mixtures comprising at least one lubricoolant oil are typically used.

Namely, the purposes of these lubricoolant mixtures are reducing wear in working tools and maintaining working tolerances.

Lubricoolant oils may be used either alone or diluted in water.

The oils that are used in the first case are known in the art as straight oils and are employed in mechanical machining operations requiring greater lubrication. Straight oils may be derived from either petroleum or vegetable oils and may be used in mixtures containing such oils with polar and/or chemically active additives.

The oils that are used in the latter case are known in the art as "water-based oils". Water-based oils are employed in mechanical machining operations that have considerable requirements in terms of cooling and final workpiece cleanness. Water-based oils are usually added with other compounds that act as emulsifiers, corrosion inhibitors, biocides, pH adjusters, antifoaming agents, dispersion aids, moisteners.

Nevertheless, the lubricoolant mixtures that comprise the aforementioned oils are subject to inevitable degradation of their chemical and physical properties due to the various agents that act thereupon during use of the machine tools. At the end of each working cycle, these mixtures are collected in a recovery tank and reused in the subsequent cycle.

As the number of cycles increases, the chemical and physical properties of mixtures are progressively degraded due to three main reasons: contamination by other lubricating oils, such as those that lubricate the systems for moving the axes of the machine tools, water evaporation in case of water-based mixtures and, finally, bacterial contamination of the mixture.

Concerning the first and second reason, the effect of mixture contamination by other oils from other parts of the machine is a reduced concentration of the lubricoolant oil in question in said mixture. Such concentration is typically monitored by withdrawing a mixture sample from the recovery tank and analyzing the later using a refractometer.

As for the third reason, the effect of a bacterial contamination of the mixture is a change of the pH of the mixture, as well as the presence of nitrates and sulfides therein.

For this purpose, pH measurements are made using litmus paper or digital pH meters. Systems are known in the art, such as the system as disclosed in patent application US5224051 A, that afford real-time monitoring of the pH and of any biological activity in the lubricoolant mixture. Nevertheless, these systems do not enable measurement of the lubricoolant oil contained in the mixture.

Monitoring of the concentration of the lubricoolant oil in the mixture is very important as it allows assessment of contamination of other substances that may be highly toxic for machine tool operators.

The paper "Real Time Monitoring and Automatic Regulation system for metalworking fluids (Gerulova et al, Research Papers Faculty of Material Science and Technology Slovak University of Technology, vol. 24, no. 38, 4 April 2016, pages 27-34, doi: 10.1515/rput-2016-0035) discloses a system for monitoring and adjusting a lubricoolant mixture in which concentration, pH, conductivity and temperatures are measured. The respective sensors are housed in a special tank, distinct from the recovery tank of the machine, and are independent and separate from each other. The concentration of lubricoolant oils is measured using a refractometer.

10 The paper "Oil Concentration Measurement in Metalworking Fluids by Optical Spectroscopy" (Grossi et al., Procedia Environmental Science, Engineering and Management, 5 November 2014) discloses a spectroscopic lubricoolant mixture.

The paper "Determination of oil concentration in soluble oil cutting fluids using near-infrared spectroscopy" (Carduner K R et al., Process Control and Quality 1992 FEB, vol. 2, no. 2, February 1992, pages 143-154) provides an example of near-infrared spectroscopic analysis for cutting fluids used for aluminum machining.

### Object of the Invention

Therefore, the object of the present invention is to provide an alternative system that allows automatic, real-time monitoring of the concentration of the lubricoolant oil.

A further object of the invention is to provide a reliable monitoring system, providing measures adapted to represent the conditions of the mixture as actually used by the machine tools, without affecting the operation of the various measuring parts.

These objects are fulfilled by the present invention by means of a monitoring system that comprises a detection unit submerged in the recovery tank in which a machine tool is also placed, such detection unit being equipped with means for measuring the concentration of the lubricoolant oil, which are configured to generate at least one first signal representative of the concentration measure.

Concentration is measured by means of a laser-photodiode pair, placed on two walls of the detection unit.

The two walls face each other and toward a detection seat containing a predetermined volume of lubricoolant mixture.

According to the invention, the seat is defined by a U-shaped sealed enclosure, between the two legs of the U-shape.

Since the laser and the photodiode are placed on the two sides of a fixed seat, the infrared radiation emitted by the laser always follows a constant-length path in the mixture, without placing each time a different sample in an instrument such as a spectroscope, and this provides consistent measures.

Means are further provided for transmitting such signal to a processing unit located outside the tank and connected by suitable wiring to the detection unit.

The processing unit receives and digitizes the measure signals acquired by the detection unit. Preferably, such processing unit is also equipped with means for wireless transmission of the acquired data to a computer or remote database.

Since signals are digitized outside the lubricoolant mixture, space may be saved in the sealed enclosure, thereby protecting the electronic parts of the mixture.

Optionally, in order to address the problem of noise that might affect the analog signal transmitted in the wiring between the detection unit and the processing unit, the detection unit includes a power circuit.

The contamination of other oils and their additives, as well as bacterial contamination also has the synergistic effect of producing highly carcinogenic substances. Particularly, the simultaneous presence of nitrates generated by microbial activity in the recovery tank and amines derived from contamination of the oil in question with other oils and their additives, such as corrosion inhibitors, pH adjusters or surfactants, as well as the additives themselves in the lubricoolant mixtures, result in the formation of N-nitrosamines, which are highly hazardous chemicals for operators.

Therefore, a second object of the present invention is to provide a system that allows simultaneous, real-time monitoring of both oil concentration and, if any, bacterial activity in the mixture.

This object is fulfilled by the present invention by means of a system that comprises a detection unit which is equipped with means for measuring the concentration of lubricoolant oil in the mixture, means for measuring the pH of the mixture, as well as means for measuring the electrical conductivity of the mixture. Of course, these measure signals are also transmitted to a processing unit located outside the recovery tank, which digitizes them and is in turn equipped with means for wireless transmission to a remote computer or database.

These and other objects will be more apparent from the following detailed description of a few preferred embodiments of the present invention, which shall be intended by way of example of and without limitation to the more general principles as claimed.

### BRIEF DESCRIPTION OF THE FIGURES

The following description relates to the accompanying figures, in which:
- Figure 1 is a perspective view of a machine tool, particularly a lathe, and the monitoring system of the present invention, the detection unit of said monitoring system being submerged in the recovery tank of said machine tool;
- Figure 2a is a perspective view of a detection unit of a first embodiment of the present invention;
- Figure 2b is a section of the detection unit of the first embodiment of the present invention;
- Figure 3a is a perspective view of a detection unit of a second embodiment of the present invention;
- Figure 3b is a section of the detection unit of the second embodiment of the present invention;
- Figure 4a is a perspective view of a detection unit of a third embodiment of the present invention;
- Figure 4b is a section of the detection unit of the third embodiment of the present invention;
- Figure 5a is a perspective view a detection unit of the preferred embodiment of the invention; and
- Figure 5b is a sectional view of the detection unit of the preferred embodiment of the invention.

### DETAILED DESCRIPTION

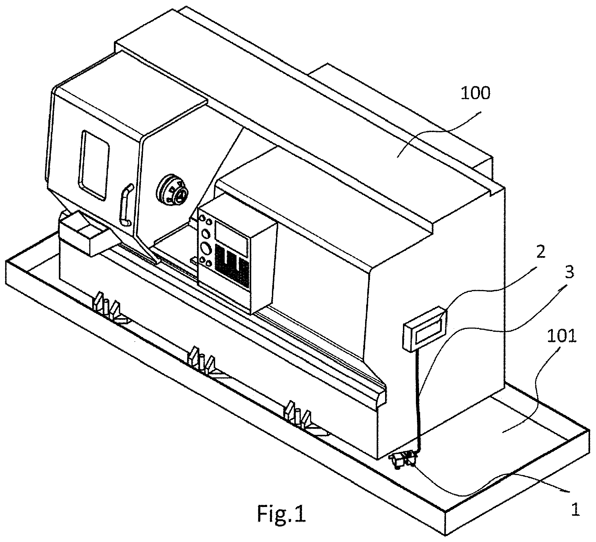

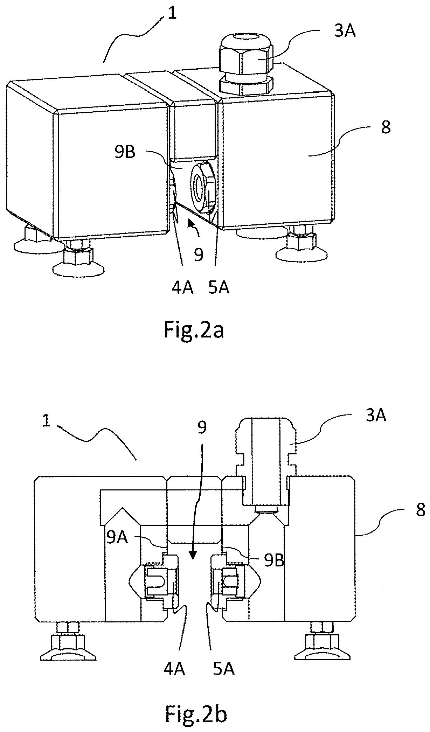

Referring to Figures 1, 2a and 2b, a first embodiment of the present invention consists of a system for monitoring the quality of the lubricoolant mixtures of the machine tools by measuring the concentration of the lubricoolant oil in said mixture.

Particularly, a machine tool, designated by numeral 100, is configured to carry out a predetermined working cycle on a workpiece, preferably a metal workpiece.

A recovery tank 101 is associated with the machine tool 100.

The recovery tank 101 contains a lubricoolant mixture.

As used herein, the term "lubricoolant mixture" is intended as a mixture comprising at least one lubricoolant oil selected from petroleum derivatives or vegetable oils. The lubricoolant oils may be either straight oils, or non water-soluble oils, or "water-based oils", or water-emulsifiable oils. Petroleum-derived lubricoolant oils are oils whose main components are synthetic hydrocarbons (e.g. Poly-alpha-olefins), ethylene/propylene oxide polymers (e.g. polyalkylene glycols, or PAG), synthetic esters (e.g. trimethyl propane troleate, di(2-ethyl-syl) adipate, 2-ethyl hexyl oleate, complex and/or polymeric esters), etc. The lubricoolant mixture may comprise certain additives such as anionic or nonionic composites, liquids, which are used for their ability to be easily mixed as emulsifiers, corrosion inhibitors, lubricants, biocides, pH adjusters, antifoaming agents, dispersion aids, moisteners. Examples of additives include: fatty acids, fatty acid akanolamides, esters, sulphonates, soaps, ethoxylated emulsifiers, chlorinated paraffins, sulphurated oils and fats, glycol esters, ethanolamines, PAG, sulphurated oils, fatty oils, biocides and fungicides. The lubricoolant mixture may also obviously comprise water if emulsifiable oils are used.

The machine tool 100 comprises pumping means which are configured to withdraw the mixture from the recovery tank 101.

Then, the mixture pumped by the machine tool 100 may then be applied by the machine tool 100, e.g. via a sprayer, to the workpiece being worked or a part of the machine tool 100 itself.

Once it has been applied, the mixture drips from the machine tool 100 or the workpiece into the recovery tank 101.

The monitoring system for monitoring the quality of the lubricoolant mixture comprises a detection unit 1 submerged in the lubricoolant mixture in the recovery tank 101 and a processing unit 2 located outside the recovery tank 101, e.g. fixed to a wall of the machine tool 100.

The detection unit 1 and the processing unit 2 are in mutual electric signal communication, i.e. electrically connected by means of suitable wiring 3.

The detection unit 1 is generally configured to generate at least one measure signal associated with the quality of the lubricoolant mixture.

Since the detection unit 1 is placed proximate to the recovery tank 101 in which the machine tool 100 is also located, the measure that is taken is highly accurate, as it reflects the chemico-physical characteristics of the mixture that is used at each cycle by the machine tool 100 itself.

A continuous and reliable monitoring of such parameters is thereby provided.

In order to protect the electronic parts required for the measurements and the conditioning thereof from the cooling lubricant mixture, the detection unit 1 comprises a sealed enclosure 8 in which such components are housed.

The wiring 3 sealingly fits into the enclosure 8 using a cable gland 3A.

In the first embodiment, the detection unit 1 comprises:
- means for measuring the concentration 4, 5 of the lubricoolant oil in the mixture, such measuring means being configured to generate a first signal representative of the concentration measure; and
- means for transmitting the at least one measure signal, which coincides, in this embodiment, with the first signal only, to the processing unit 2 through the wiring 3.

In order to guarantee the effectiveness of the measurements, the detection unit 1 is designed to comprise first and second walls 9A, 9B in opposed and facing relationship, with a laser 4 associated with the first wall 9A and configured to emit infrared radiation, and a photodiode 5 associated with the second wall 9B, said photodiode 5 being configured to absorb the infrared radiation emitted by the laser 4 and outcoming from the predetermined volume of lubricoolant mixture, and to generate the first signal as a function of the radiation that has been absorbed.

Therefore, the control unit is configured with the two walls 9A and 9B in opposed and facing relationship, for the laser and the photodiode to face each other.

Namely, according to the invention, the detection unit 1 comprises a box-like enclosure 8 having a predetermined volume and comprises a detection seat 9 formed outside the enclosure 8 but preferably within its theoretical volume. Such detection seat 9, formed outside the enclosure 8, is occupied by a predetermined volume of lubricoolant mixture.

In other words, the seat 9 of the control unit 1 is formed in such a manner that the laser and the photodiode will always target, through the various detections, the same mixture volume.

For this purpose, the two walls 9A and 9B shall be situated at a such a distance from each other that the laser beam will pass through a sufficient portion of mixture, but the photodiode will not be so far from the laser as to generate detrimental noise. An effective distance between such two walls 9A and 9B was particularly found to range from a few centimeters to a few tens of centimeters.

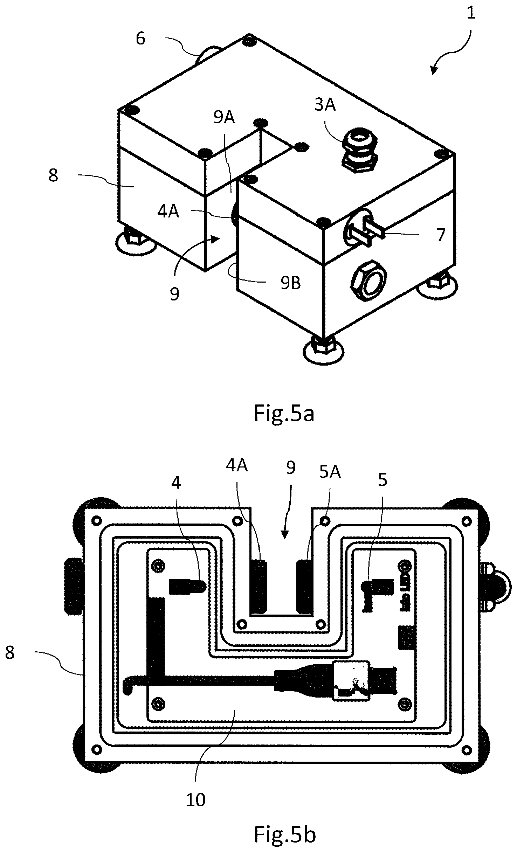

As shown in Figures 5a and 5b, the detection seat 9 is open on one side, and the first and second walls 9A, 9B are the two mutually facing and opposed walls of the seat 9.

The means 4, 5 for measuring the concentration of the lubricoolant oil further comprise a laser 4 associated with the first wall 9A and a photodiode 5 associated with the second wall 9B.

Therefore, since the first and second walls 9A, 9B are in mutually facing and opposed relationship, then the beam emitted by the laser and received by the photodiode shall always pass through the same volume of lubricoolant mixture, thereby ensuring constant system monitoring.

According to the invention the enclosure 8 has a U shape, and the detection seat 9 is defined between the two legs of the U shape.

The first and second walls 9A, 9B define the inner faces of the two legs of the U shape.

It shall be noted that:
- the laser 4 is preferably of vertical-cavity surface-emitting semiconductor type, and is configured to emit infrared radiation, preferably at 850 nm.
- the photodiode 5 is configured to absorb the infrared radiation emitted by the laser 4 (outcoming from the predetermined volume of lubricoolant mixture), and to generate the first signal as a function of the radiation that has been absorbed, for instance as a current signal proportional to the radiation absorbed.

The photodiode 5 is preferably of the silicon p-i-n type.

The laser 4 and the photodiode 5 are optically coupled through the detection seat 9.

More in detail, as particularly shown in Figures 5a, 5b, the enclosure 8 has two transparent elements 4A, 5A associated with the first and second walls 9A, 9B respectively.

The laser 4 is housed in the enclosure 8 behind the transparent element 4A of the first walls 9A, and the photodiode 5 is housed in the enclosure 8 behind the transparent element 5A of the second wall 9B.

Therefore, the radiation emitted by the laser 4 passes through the two transparent elements 4A, 5A, thereby ensuring optical communication with the photodiode 5 with no sealing problem.

The processing unit 2 comprises means for receiving and digitizing the at least one measure signal and a wireless transmitter configured for interaction with a remote computer or database.

Particularly, the signal will be digitized by the processing unit 2, i.e. outside the mixture, rather than by the detection unit 1. By this arrangement, the number of parts to be disposed in the enclosure 8 for protection from the lubricoolant mixture, and hence the size the enclosure 8 itself may be reduced.

Then, the measure signal is transmitted through the wiring 3 in analog form. Preferably, the detection unit 1 comprises an amplifying circuit 10 configured to amplify the measure signal in order to provide an amplified measure signal to the processing unit 2 through the wiring 3.

The transmission of an amplified signal directly from the detection unit 1 can prevent the small signal generated by the photodiode 5 from being transmitted through the wiring 3, thereby providing protection from typical noise signals of an industrial environment.

For this purpose, the amplification circuit 10 may comprise, for example, a transimpedance amplifier connected to the photodiode 5.

This is found to be particularly advantageous, as the processing unit 2 may be often very far from the recovery tank in the industrial environments in which the machine tools as described herein are located. In this context, the length of the wiring 3 will affect the quality of the signal generated by the photodiode 5 due to attenuation caused the wiring itself and/or to the electromagnetic noise that is found in industrial environments.

It shall be noted that the remote computer or database are in wireless communication with the processing unit 2, and the latter is configured to receive the at least one measure signal from the wireless transmitter and analyze the at least one measure signal, and to generate a status signal representative of the quality of the lubricoolant mixture.

For example, the signal may be representative of the need to replace or top up the mixture, or the possibility to perform additional work cycles without such replacement.

The status signal may be made visible to the user directly at the remote computer, or at the processing unit 2, by being wiressly transmitted thereto.

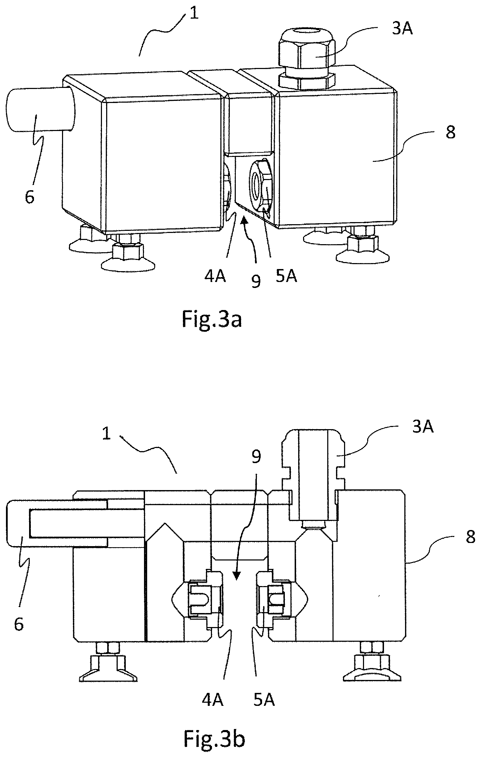

Referring to figures 1, 3a and 3b, the detection unit 1 comprises means for measuring the pH 6 of the mixture, which are configured to generate a second signal representative of the measure of pH. It shall be noted that the pH measuring means 6 comprise a glass electrode.

Therefore, the second signal may be given by a current signal proportional to the concentration of ions H⁺ in the mixture.

The second signal is also designed to be amplified by the amplifier 10.

Particularly, the amplification of the first and of the second signals may be obtained by a single circuit or distinct circuits.

In one embodiment all the signals are amplified using distinct amplifiers that are fixed to a common support, such as a printed circuit.

Likewise, the digitizing means in the processing unit 2 can digitize the first and the second signals together or separately.

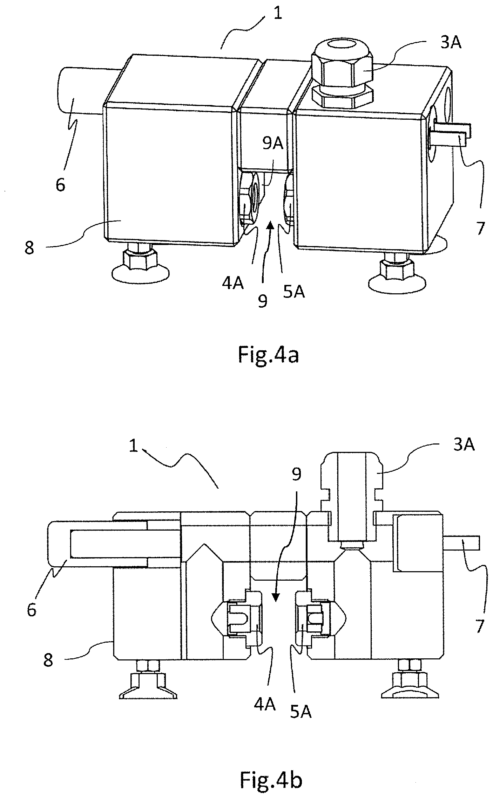

Referring to figures 1, 4a and 4b, the detection unit 1 comprises means for measuring the electrical conductivity 7 of the mixture, which are configured to generate a third signal representative of the electrical conductivity measure.

The means 7 for measuring conductivity comprise two electrodes, preferably made of stainless steel, and a calibration resistor, said electrodes forming a voltage divider with said resistor.

The means for digitizing said first, second and third signals are composed of an analog-to-digital converter.

The invention disclosed herein fulfills the intended objects and overcomes the limitations of the prior art.

The simultaneous provision of means for real-time monitoring of both the concentration of the lubricoolant oil and the bacterial activity, if any, provides a system that affords continuous control of the oil quality, as well as early detection of any oil degradation that can make oil potentially carcinogenic or otherwise harmful for the operators of the machine tools, and as a result allows oil replacement before the occurrence of such drawbacks. Such control is fully automated and requires no manual action, particularly no need to withdraw a fluid sample for laboratory analysis. The characteristics of lubricoolant mixture are analyzed directly in the recovery tank of the machine tool and the measure data, once digitized, are wirelessly sent to a remote computer, in which they can be analyzed in real time by means of appropriate software.

## Claims

1. A system for monitoring an oil concentration in a lubricoolant mixture for a machine tool (100) comprising:
- a machine tool (100);
- a recovery tank (101) containing a lubricoolant mixture comprising at least one lubricoolant oil, the machine tool (100) being configured to withdraw the lubricoolant mixture directly from the recovery tank (101);
- a detection unit (1) configured to generate at least one measure signal;
- a processing unit (2) located outside the recovery tank (101); and
- a wiring (3) connecting the detection unit (1) and the processing unit (2), the processing unit (2) being configured to receive said at least one measure signal through the wiring (3) and to digitize it;
- said detecting unit (1) comprises means (4, 5) for measuring a concentration of the at least one lubricoolant oil in the lubricoolant mixture, which are configured to generate a first measure signal representative of the measure of said concentration,
**characterized in that** said detection unit (1) is submerged in the lubricoolant mixture within the recovery tank (101) of the machine tool (100) and said detection unit (1) comprises:
- a U-shaped sealed enclosure (8) having two legs with inner faces, which are defined by first and second mutually opposed and facing walls (9A, 9B), a detection seat (9) being defined between said two legs for containing a predetermined volume of the lubricoolant mixture, said enclosure having two transparent elements (4A, 5A) associated with the first and second walls (9A, 9B) respectively,
- a laser (4) which is housed in the U-shaped sealed enclosure (8) behind the transparent element (4A) of the first wall (9A), and is configured to emit an infrared radiation, and
- a photodiode (5) housed in the U-shaped sealed enclosure (8) behind the transparent element (5A) of the second wall (9B), the photodiode (5) being configured to absorb the infrared radiation emitted by the laser (4) outcoming from said predetermined volume of the lubricoolant mixture and to generate the first measure signal as a function of the infrared radiation that has been absorbed.

2. A monitoring system as claimed in claim 1, wherein said laser (4) is a vertical-cavity surface-emitting semiconductor laser, configured to emit said infrared radiation toward said photodiode (5) and the photodiode (5) is a silicon p-i-n photodiode (5).

3. A monitoring system (4) as claimed in claim 2, wherein said laser (4) is a 850 nm laser.

4. A monitoring system as claimed in any of claims 1 to 3, wherein the detection unit (1) comprises an amplifying circuit (10) configured to amplify the at least one measure signal generated by the detection unit (1) thereby providing an amplified measure signal to the processing unit (2) through the wiring (3).

5. A monitoring system as claimed in claim 4, in which the amplifying circuit (10) comprises a transimpedance amplifier connected to said photodiode (5).

6. A monitoring system as claimed in any claim 1 to 5, wherein the machine tool (100) is housed in the recovery tank (101).

7. A monitoring system as claimed in any of the preceding claims, comprising a remote database, wherein said processing unit (2) comprises a wireless transmitter configured for interaction with said remote database.

8. A monitoring system as claimed in claim 7, wherein the remote database is configured to receive the at least one measure signal from the wireless transmitter and analyze it, and to generate a status signal representative of the quality of the lubricoolant mixture.

9. A monitoring system as claimed in any of claims 1 to 8, wherein the detection unit (1) comprises means for measuring the pH (6) of the lubricoolant mixture which are configured to generate a second measure signal representative of the measurement of said pH.

10. A monitoring system as claimed in claim 9, wherein said means for measuring the pH (6) comprise a glass electrode.

11. A monitoring system as claimed in any of claims 1 to 10, wherein said detection unit (1) comprises means for measuring the electrical conductivity (7) of the lubricoolant mixture which are configured to generate a third measure signal representative of the measurement of said electrical conductivity.

12. A monitoring system as claimed in claim 11, wherein said means (7) for measuring the electrical conductivity comprise at least two electrodes and a calibration resistor, said at least two electrodes forming a voltage divider with said calibration resistor.

13. A monitoring system as claimed in claim 12 wherein said at least two electrodes are made of stainless steel.

## Patentansprüche

1. System zum Überwachen einer Ölkonzentration in einem Kühlschmierstoffgemisch für eine Werkzeugmaschine (100), umfassend:
- eine Werkzeugmaschine (100);
- einen Rückgewinnungstank (101), der ein Kühlschmierstoffgemisch enthält, umfassend mindestens ein Kühlschmieröl, wobei die Werkzeugmaschine (100) konfiguriert ist, um das Kühlschmierstoffgemisch direkt aus dem Rückgewinnungstank (101) abzulassen;
- eine Erfassungseinheit (1), die konfiguriert ist, um mindestens ein Messsignal zu erzeugen;
- eine Verarbeitungseinheit (2), die sich außerhalb des Rückgewinnungstanks (101) befindet; and
- eine Verdrahtung (3), die die Erfassungseinheit (1) und die Verarbeitungseinheit (2) verbindet, wobei die Verarbeitungseinheit (2) dazu konfiguriert ist, das mindestens eine Messsignal über die Verdrahtung (3) zu empfangen und dieses zu digitalisieren;
- wobei die Erfassungseinheit (1) Mittel (4, 5) zur Messung einer Konzentration des mindestens einen Kühlschmieröls im Kühlschmierstoffgemisch umfasst, welche konfiguriert sind, um ein erstes für die Messung der Konzentration repräsentatives Messsignal zu erzeugen,
**dadurch gekennzeichnet, dass** die Erfassungseinheit (1) im Kühlschmierstoffgemisch im Inneren des Rückgewinnungstanks (101) der Werkzeugmaschine (100) eingetaucht ist und, dass die Erfassungseinheit (1) Folgendes umfasst:
- ein U-förmiges abgedichtetes Gehäuse (8), das zwei Schenkeln mit Innenflächen aufweist, welche durch erste und zweite einander gegenüberliegende und zugewandte Wände (9A, 9B) definiert sind; einen Erfassungssitz (9), der zwischen beiden Schenkeln definiert ist, um ein vorbestimmtes Volumen des Kühlschmierstoffgemisches zu enthalten, wobei das Gehäuse zwei transparente Elemente (4A, 5A) aufweist, die jeweils der ersten und zweiten Wand (9A, 9B) zugeordnet sind,
- einen Laser (4), der in dem U-förmigen abgedichteten Gehäuse (8) hinter dem transparenten Element (4A) der ersten Wand (9A) aufgenommen und dazu konfiguriert ist, eine Infrarotstrahlung zu emittieren, und
- eine Fotodiode (5), die in dem U-förmigen abgedichteten Gehäuse (8) hinter dem transparenten Element (5A) der zweiten Wand (9A) aufgenommen ist, wobei die Fotodiode (5) dazu konfiguriert ist, die von dem Laser (4) emittierte Infrarotstrahlung zu absorbieren, die aus dem vorbestimmten Volumen des Kühlschmierstoffgemisches austritt, und das erste Messsignal als eine Funktion der absorbierten Infrarotstrahlung zu erzeugen.

2. Überwachungssystem wie in Anspruch 1 beansprucht, wobei der Laser (4) ein oberflächenemittierender Halbleiterlaser mit vertikaler Kavität ist, der dazu konfiguriert ist, die Infrarotstrahlung in Richtung der Fotodiode (5) zu emittieren, und wobei die Fotodiode (5) eine Silizium-PIN-Fotodiode (5) ist.

3. Überwachungssystem (4) wie in Anspruch 2 beansprucht, wobei der Laser (4) ein 850 nm Laser ist.

4. Überwachungssystem wie in irgendeinem der Ansprüche von 1 bis 3 beansprucht, wobei die Erfassungseinheit (1) eine Verstärkungsschaltung (10) umfasst, die dazu konfiguriert ist, das mindestens eine von der Erfassungseinheit (1) erzeugte Messsignal zu verstärken, wodurch ein verstärktes Messsignal zur Verarbeitungseinheit (2) über die Verkabelung (3) bereitgestellt wird.

5. Überwachungssystem wie in Anspruch 4 beansprucht, bei dem die Verstärkungsschaltung (10) einen Transimpedanzverstärker umfasst, der mit der Fotodiode (5) verbunden ist.

6. Überwachungssystem wie in irgendeinem der Ansprüche von 1 bis 5 beansprucht, wobei die Werkzeugmaschine (100) in dem Rückgewinnungstank (101) aufgenommen ist.

7. Überwachungssystem wie in irgendeinem der vorgehenden Ansprüche beansprucht, umfassend eine Ferndatenbank, wobei die Verarbeitungseinheit (2) einen drahtlosen Sender umfasst, der für die Interaktion mit der Ferndatenbank konfiguriert ist.

8. Überwachungssystem wie in Anspruch 7 beansprucht, wobei die Ferndatenbank dazu konfiguriert ist, das mindestens eine Messsignal vom drahtlosen Sender zu empfangen und dieses zu konfigurieren, sowie ein für die Qualität des Kühlschmierstoffgemisches repräsentatives Statussignal zu erzeugen.

9. Überwachungssystem wie in irgendeinem der Ansprüche von 1 bis 8 beansprucht, wobei die Erfassungseinheit (1) Mittel zur Messung des pH-Wertes (6) des Kühlschmierstoffgemisches umfasst, welche konfiguriert sind, um ein zweites Messsignal zu erzeugen, das für die Messung des pH-Wertes repräsentativ ist.

10. Überwachungssystem wie in Anspruch 9 beansprucht, wobei die Mittel zur Messung des pH-Wertes (6) eine Glaselektrode umfassen.

11. Überwachungssystem wie in irgendeinem der Ansprüche von 1 bis 10 beansprucht, wobei die Erfassungseinheit (1) Mittel zur Messung der elektrischen Leitfähigkeit (7) des Kühlschmierstoffgemisches umfasst, welche konfiguriert sind, um ein drittes Messsignal zu erzeugen, das für die Messung der elektrischen Leitfähigkeit repräsentativ ist.

12. Überwachungssystem wie in Anspruch 11 beansprucht, wobei die Mittel (7) zur Messung der elektrischen Leitfähigkeit mindestens zwei Elektroden und einen Kalibrierungswiderstand umfassen, wobei die mindestens zwei Elektroden mit dem Kalibrierungswiderstand einen Spannungsteiler bilden.

13. Überwachungssystem wie in Anspruch 12 beansprucht, wobei die mindestens zwei Elektroden aus rostfreiem Stahl hergestellt sind.

## Revendications

1. Système de surveillance d'une concentration d'huile dans un mélange lubrifiant réfrigérant pour une machine-outil (100) comprenant :
- une machine-outil (100) ;
- un réservoir de récupération (101) contenant un mélange lubrifiant réfrigérant comprenant au moins une huile lubrifiante réfrigérante, la machine-outil (100) étant configurée pour prélever le mélange lubrifiant réfrigérant directement du réservoir de récupération (101) ;
- une unité de détection (1) configurée pour générer au moins un signal de mesure ;
- une unité de traitement (2) localisée à l'extérieur du réservoir de récupération (101) ; et
- un câblage (3) connectant l'unité de détection (1) et l'unité de traitement (2), l'unité de traitement (2) étant configurée pour recevoir ledit au moins un signal de mesure à travers le câblage (3) et pour le numériser ;
- ladite unité de détection (1) comprend des moyens (4, 5) pour mesurer une concentration de l'au moins une huile lubrifiante réfrigérante dans le mélange lubrifiant réfrigérant, qui sont configurés pour générer un premier signal de mesure représentatif de la mesure de ladite concentration,
**caractérisé en ce que** ladite unité de détection (1) est submergée dans le mélange lubrifiant réfrigérant à l'intérieur du réservoir de récupération (101) de la machine-outil (100) et ladite unité de détection (1) comprend :
- une enceinte étanche en forme de U (8) ayant deux pattes avec des faces intérieures, qui sont définies par des première et deuxième parois (9A, 9B) mutuellement opposées et tournées l'une vers l'autre, un siège de détection (9) étant défini entre lesdites deux pattes pour contenir un volume prédéterminé de mélange lubrifiant réfrigérant, ladite enceinte ayant deux éléments transparents (4A, 5A) associés avec les première et deuxième parois (9A, 9B) respectivement,
- un laser (4) qui est logé dans l'enceinte étanche en forme de U (8) derrière l'élément transparent (4A) de la première paroi (9A), et est configuré pour émettre un rayonnement infrarouge, et
- une photodiode (5) logée dans l'enceinte étanche en forme de U (8) derrière l'élément transparent (5A) de la deuxième paroi (9B), la photodiode (5) étant configurée pour absorber le rayonnement infrarouge émis par le laser (4) provenant dudit volume prédéterminé du mélange lubrifiant réfrigérant et pour générer le premier signal de mesure en fonction du rayonnement infrarouge qui a été absorbé.

2. Système de surveillance tel que revendiqué dans la revendication 1, dans lequel ledit laser (4) est un laser semi-conducteur à cavité verticale émettant par la surface, configuré pour émettre ledit rayonnement infrarouge vers ladite photodiode (5) et la photodiode (5) est une photodiode p-i-n au silicium (5).

3. Système de surveillance (4) tel que revendiqué dans la revendication 2, dans lequel ledit laser (4) est un laser de 850 nm.

4. Système de surveillance tel que revendiqué dans l'une quelconque des revendications 1 à 3, dans lequel l'unité de détection (1) comprend un circuit d'amplification (10) configuré pour amplifier l'au moins un signal de mesure généré par l'unité de détection (1) en fournissant ainsi un signal de mesure amplifié à l'unité de traitement (2) à travers le câblage (3).

5. Système de surveillance tel que revendiqué dans la revendication 4, dans lequel le circuit d'amplification (10) comprend un amplificateur à transimpédance connecté à ladite photodiode (5).

6. Système de surveillance tel que revendiqué dans l'une quelconque des revendications 1 à 5, dans lequel la machine-outil (100) est logée dans le réservoir de récupération (101).

7. Système de surveillance tel que revendiqué dans l'une quelconque des revendications précédentes, comprenant une base de données distante, dans lequel ladite unité de traitement (2) comprend un émetteur sans fil configuré pour une interaction avec ladite base de données distante.

8. Système de surveillance tel que revendiqué dans la revendication 7, dans lequel la base de données distante est configurée pour recevoir l'au moins un signal de mesure de l'émetteur sans fil et l'analyser, et pour générer un signal d'état représentatif de la qualité du mélange lubrifiant réfrigérant.

9. Système de surveillance tel que revendiqué dans l'une quelconque des revendications 1 à 8, dans lequel l'unité de détection (1) comprend des moyens pour mesurer le pH (6) du mélange lubrifiant réfrigérant qui sont configurés pour générer un deuxième signal de mesure représentatif de la mesure dudit pH.

10. Système de surveillance tel que revendiqué dans la revendication 9, dans lequel lesdits moyens pour mesurer le pH (6) comprennent une électrode de verre.

11. Système de surveillance tel que revendiqué dans l'une quelconque des revendications 1 à 10, dans lequel l'unité de détection (1) comprend des moyens pour mesurer la conductivité électrique (7) du mélange lubrifiant réfrigérant qui sont configurés pour générer un troisième signal de mesure représentatif de la mesure de ladite conductivité électrique.

12. Système de surveillance tel que revendiqué dans la revendication 11, dans lequel lesdits moyens (7) pour mesurer la conductivité électrique comprennent au moins deux électrodes et une résistance d'étalonnage, lesdites au moins deux électrodes formant un diviseur de tension avec ladite résistance d'étalonnage.

13. Système de surveillance tel que revendiqué dans la revendication 12, dans lequel lesdites au moins deux électrodes sont réalisées en acier inoxydable.
